# EUROPEAN PATENT SPECIFICATION

(11) **EP 0 360 829 B1**
(45) Date of publication and mention of the grant of the patent: **26.05.1993**
(21) Application number: 88905141.3
(22) Date of filing: 25.05.1988
(51) Int. Cl.: C07C 17/34, C07C 25/24

(54) **DEHYDROBROMINATION OF SUBSTITUTED ALPHA-HALOCUMENE**
DEHYDROBROMIERUNG VON SUBSTITUIERTEM ALPHA-HALOCUMOL
DEHYDROBROMINATION D'ALPHA-HALOCUMENE SUBSTITUE

(30) Priority: 26.05.1987 US 53924
(43) Date of publication of application: 04.04.1990
(73) Proprietor: DOWELANCO, Indianapolis, Indiana 46268-1189 (US)
(72) Inventor: JOHNSON, Mark, R., Grayslake, IL 60030 (US)
(74) Representative: Smulders, Theodorus A.H.J., Ir.
(86) International application number: US8801735
(87) International publication number: WO8809319

(56) References cited:
- GB-A- 792 860

## Description

The present invention relates to processes for preparing substituted α-methylstyrenes.

Substituted α-methylstyrenes are compounds useful as intermediates for preparing pesticides, such as herbicides and insecticides, and elastomer compositions.

U.S. Patent 3,067,182 describes a process for preparing isopropenylbenzyl chloride in which isopropylbenzyl chloride (89 percent para-, 11 percent ortho) is chlorinated, and the chlorinated material is distilled at a boiling point of 73°C to 74°C at 0.01 to 0.05 millimeters of mercury pressure absolute (1.33 to 6.67 Pa) to yield isopropenylbenzyl chloride. Inherently, this process operates at very low, sub-atmospheric pressures. In order to provide such low pressures, fairly sophisticated and expensive pressure reduction vessels are used.

U.S. Patent 1,687,903 describes a process wherein styrols are prepared from chlorethylbenzols by heating the chlorethylbenzols to decomposition temperatures ranging from 500-725°C. Clearly, such a process has the disadvantage of large energy requirements to maintain such high temperatures.

U.S. Patent 4,594,467 describes a process wherein 3,5-dichloro-α-methylstyrene is prepared by dehydrobromination of α-bromo-3,5-dichlorocumene, in an alkaline medium at a temperature above 70°C, using a quaternary ammonium phase-transfer catalyst. Processes utilizing a caustic or basic medium, such as sodium hydroxide (NaOH) are incompatible with reaction vessels which are glass lined.

While some of these processes may generally achieve good yields, they have the disadvantage of requiring the use of expensive and often toxic catalysts which must be removed at the completion of the reaction. This separation of the catalyst from the reaction mixture and its disposal often requires the use of multiple handlings. After separation, the phase transfer catalyst requires waste disposal and/or recycling procedures using elaborate and expensive recycling apparatus. It would clearly be desirable to provide a process which makes unnecessary the need to utilize catalysts.

The present invention is directed to a process for preparing a substituted α-methylstyrene compound of the formula: 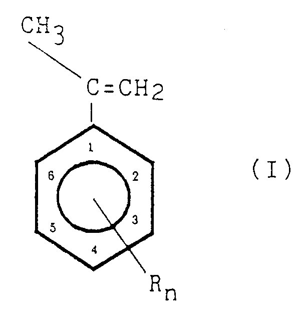
wherein R represents halogen, an alkyl group having from 1 to 6 carbon atoms, an alkoxy group having from 1 to 6 carbon atoms or trifluoromethyl and n represents 0, 1, 2, 3, 4 or 5; characterized by
(a) heating, under ambient pressures, a mixture of water and α-halocumene compound of the formula: 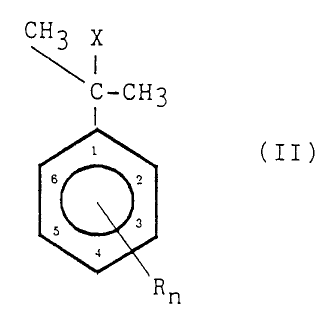 wherein X is chloro, bromo or iodo; to a temperature ranging between ambient to 130°C, effective to convert the α-halocumene compound to α-methylstyrene compound; and
(b) separating the α-methylstyrene compound formed therein from said mixture.

In the desired α-methylstyrene of Formula (I), preferably R is chloro and n is 2. Also, preferred is that R is substituted in the 3- and 5-positions on the phenyl ring. Most preferably, R is chloro, n is 2 and R is substituted at the 3- and 5-positions on the ring, a compound known as 3,5-dichloro-α-methylstyrene (DCAMS).

Regarding the α-halocumene starting material of Formula (II), preferably X is bromo. Also preferred, is that R is chloro and n is 2. Also preferred is that R is substituted in the 3- and 5-position on the ring. Most preferably, X is bromo, R is chloro, n is 2 and R is substituted at the 3- and 5-position on the ring, a compound known as α-bromo-3,5-dichlorocumene (BrDCC).

The present invention has the advantage of preparing substituted α-methylstyrenes without the need for catalysts, while providing substituted α-methylstyrenes in yields and purity as good or better than that described by other known methods.

The heating of the α-halocumene starting material of Formula II with water, and optionally with an organic solvent, is carried out at temperatures ranging from ambient to 130 degrees Centigrade (°C), preferably from 95° to 115°C.

Optionally, the reaction mixture may also contain a liquid inert organic solvent in an amount effective to at least dissolve the α-halocumene compound of Formula II so as to improve handling of this starting material. Representative inert organic solvents suitable for dissolving the α-halocumene compound include but are not limited to halogenated aromatic and aliphatic hydrocarbons such as monochlorobenzene, chloroform, carbon tetrachloride; or non-halogen containing aromatic or aliphatic hydrocarbons such as benzenes, xylenes, hexane, cyclohexane, pentanes, etc. Generally, the inert organic solvent can be present in an amount ranging from 0 to 2 parts by weight inert organic solvent to 1 part by weight α-halocumene, preferably from 0 to 1 part by weight inert organic solvent.

Water, at least equivalent in quality to that used for industrial purposes, can be used in the process of the present invention. The amount of water in association with the α-halocumene of Formula II should be an amount effective to promote dehydrohalogenation of said α-halocumene. Generally, water is present in an amount ranging from 1 to 4 parts by weight α-halocumene to one part by weight water, preferably from one part water to 1.1 to 1 part by weight α-halocumene.

The reaction mixture of water and α-halocumene may contain minor amounts of impurities such as metal or base contaminants. Such impurities may be present in amounts which allow the process to operate substantially the same way to give substantially the same results as if the process were run using a mixture which does not possess added or inherent impurities.

The heating of the α-halocumene and water, and optionally, organic solvent should be maintained for a period effective to convert the α-halocumene to α-methylstyrene, preferably from 1 to 24 hours, more preferably from 2 to 6 hours. The reaction mixture should be stirred during the course of the heating.

Separation to recover the desired α-methylstyrene compound of Formula I from the reaction mixture can be accomplished by phase separation of the aqueous and organic layers, i.e., decanting the aqueous layers. The decanted organic layer containing the desired α-methylstyrene compound can be treated by conventional procedures, such as stripping, distillation, or crystallization.

The following examples illustrate the present invention in a manner by which it can be practiced.

### Example 1

A sample of 273.2 g of crude α-bromo-3,5-dichlorocumene (BrDCC-62.4 percent purity) with the balance monochlorobenzene (MCB) was treated with 100 milliliters (ml) water, and the mixture was stirred and heated at 100-105 degrees Centigrade (°C) for 3.5 hours. Phase inversion of the water and the organic layer occurred after 1 hour. After 3.5 hours an additional 50 ml water was added, and the mixture was heated an additional 2 hours. The mixture was allowed to cool, the phases were separated by decantation, the product phase was washed with an additional 25 ml water, and the aqueous phase was extracted with an additional 10 ml monochlorobenzene (MCB). The crude product weighs 225.4 g and contains 50.8 percent 3,5-dichloro-α-methylstyrene (DCAMS-96 percent yield). Distillation was done through a short vigreaux column. Monochlorobenzene was removed at 15-20 millimeters (mm) mercury (Hg) (2000 to 2666 Pa) at an overhead temperature of 35°C. The fraction containing 3,5-dichloro-α-methylstyrene was taken at an overhead temperature of 108-110°C. The product cut contains a 94 percent yield of 3,5-dichloro-α-methylstyrene.

### Example 2

A sample of 124.3 g of crude solvent-free α-bromo-3,5-dichlorocumene (BrDCC-91 percent purity) and 93 ml of water were stirred and heated at 100-110°C for 3 hours. The mixture was allowed to cool, the phases were separated by decantation, and the product phase washed with 10 ml water. The product was distilled through a short vigreaux column under vacuum with an overhead temperature of 157-160°C and the pot temperature was maintained at 155-200°C. The overheads contain a 95 percent yield of 3,5-dichloro-α-methylstyrene.

## Claims

1. A process for preparing a substituted α-methylstyrene compound of the formula: 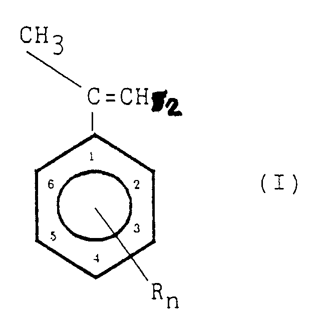 wherein R represents halogen, an alkyl group having from 1 to 6 carbon atoms, an alkoxy group having from 1 to 6 carbon atoms or trifluoromethyl and n represents 0, 1, 2, 3, 4 or 5; characterized by
(a) heating, under ambient pressures, a mixture of water and α-halocumene compound of the formula: 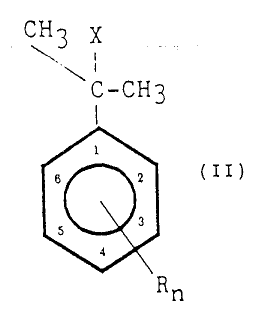 wherein X is chloro, bromo or iodo; to a temperature ranging between ambient to 130°C, effective to convert the α-halocumene compound to α-methylstyrene compound; and
(b) separating the α-methylstyrene compound formed therein from said mixture.

2. The process of Claim 1 wherein the reaction mixture is heated to a temperature ranging between 95 to 115 degrees Centigrade.

3. The process of Claim 1 wherein water is present in an amount ranging from 1 to 4 parts by weight α-halocumene compound to 1 part by weight water.

4. The process of Claim 1 wherein the water is present in an amount ranging from 1.1 to 1 part by weight α-halocumene compounds to 1 part by weight water.

5. The process of Claim 1 wherein the reaction mixture of water and the α-halocumene compound also contains an inert organic solvent in an amount effective to dissolve said α-halocumene compound.

6. The process of Claim 5 wherein the inert organic solvent is a halogenated aromatic or aliphatic hydrocarbon.

7. The process of Claim 6 wherein the inert solvent is monochlorobenzene, chloroform or carbon tetrachloride.

8. The process of any one of Claims 1 to 7 wherein the α-halocumene compound of Formula II, R is chloro, X is bromo and n is 0, 1 or 2.

9. The process of any one of Claims 1 to 7 where in the α-halocumene compound is α-bromo-3,5-dichlorocumene.

## Patentansprüche

1. Verfahren zur Herstellung einer substituierten α-Methylstyrol-Verbindung der Formel: 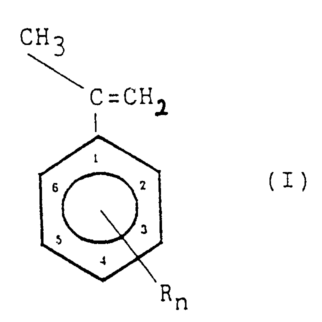 wobei R ein Halogen, eine Alkylgruppe mit 1 bis 6 C-Atomen, eine Alkoxygruppe mit 1 bis 6 C-Atomen oder Trifluormethyl darstellt und n 0, 1, 2, 3, 4 oder 5 ist;
dadurch gekennzeichnet, daß
(a) eine Mischung aus Wasser und einem α-Halogencumol der Formel: 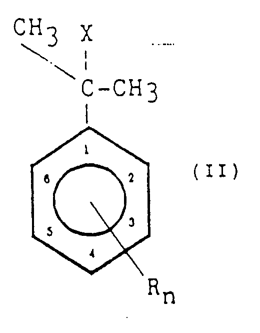 wobei X Chlor, Brom oder Iod ist bei Normaldruck auf eine Temperatur von zwischen Raumtemperatur und 130°C, welche zur Umwandlung der α-Halogencumolverbindung zu α-Methylstyrol wirksam ist, geheizt wird; und
(b) die darin gebildete α-Methylstyrol-Verbindung von der Mischung getrennt wird.

2. Verfahren nach Anspruch 1, worin die Reaktionsmischung auf eine Temperatur von zwischen 95 und 115°C geheizt wird.

3. Verfahren nach Anspruch 1, worin Wasser in einer Menge von 1 Gewichtsteil Wasser auf 1 bis 4 Gewichtsteile α-Halogencumol vorhanden ist.

4. Verfahren nach Anspruch 1, worin das Wasser in einer Menge von 1 Gewichtsteil Wasser auf 1,1 bis 1 Gewichtsteil α-Halogencumol vorhanden ist.

5. Verfahren nach Anspruch 1, worin die Reaktionsmischung aus Wasser und der α-Halogencumolverbindung ebenfalls ein inertes organisches Lösungsmittel in einer zur Auflösung der α-Halogencumolverbindung wirksamen Menge enthält.

6. Verfahren nach Anspruch 5, worin das inerte organische Lösungsmittel ein halogenierter aromatischer oder aliphatischer Kohlenwasserstoff ist.

7. Verfahren nach Anspruch 6, worin das inerte Lösungsmittel Monochlorbenzol, Chloroform oder Tetrachlorkohlenstoff ist.

8. Verfahren nach einem der Ansprüche 1 bis 7, worin in der α-Halogencumolverbindung der Formel II R Chlor, X Brom und n O, 1 oder 2 ist.

9. Verfahren nach einem der Ansprüche 1 bis 7, worin die α-Halogencumolverbindung α-Brom-3,5-Dichlorcumol ist.

## Revendications

1. Procédé de préparation d'un α-méthylstyrène substitué de formule : 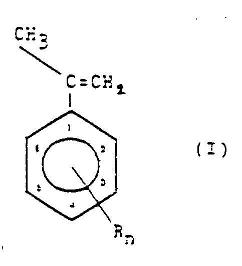 dans laquelle R représente un atome d'halogène, un groupe alkyle comportant de 1 à 6 atomes de carbone, un groupe alkoxy comportant de 1 à 6 atomes de carbone ou un groupe trifluorométhyle, et n vaut 0, 1, 2, 3, 4 ou 5,
caractérisé en ce que
a) on chauffe, sous la pression ambiante, un mélange d'eau et d'un α-halogénocumène de formule : 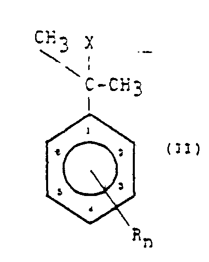 dans laquelle X représente un atome de chlore, de brome ou d'iode, à une température située entre la température ambiante et 130°C, efficace pour convertir l'α-halogénocumène en α-méthylstyrène, et
b) on sépare l'α-méthylstyrène formé du mélange réactionnel.

2. Procédé selon la revendication 1, dans lequel on chauffe le mélange réactionnel à une température située entre 95 et 115°C.

3. Procédé selon la revendication 1, dans lequel la quantité d'eau présente correspond à une proportion de 1 à 4 parties en poids d'α-halogénocumène pour 1 partie en poids d'eau.

4. Procédé selon la revendication 1, dans lequel la quantité d'eau présente correspond à une proportion de 1 à 1,1 partie en poids d'α-halogénocumène pour 1 partie en poids d'eau.

5. Procédé selon la revendication 1, dans lequel le mélange réactionnel d'eau et d'α-halogénocumène contient également un solvant organique inerte, en une quantité efficace pour dissoudre l'α-halogénocumène.

6. Procédé selon la revendication 5, dans lequel le solvant organique inerte est un hydrocarbure halogéné aromatique ou aliphatique.

7. Procédé selon la revendication 6, dans lequel le solvant inerte est du monochlorobenzène, du chloroforme ou du tétrachlorure de carbone.

8. Procédé selon l'une des revendications 1 à 7, dans lequel, dans l'α-halogénocumène de formule II, R représente un atome de chlore, X représente un atome de brome et n vaut 0, 1 ou 2.

9. Procédé selon l'une des revendications 1 à 7, dans lequel l'α-halogénocumène est de l'α-bromo-3,5-dichlorocumène.
